# EUROPEAN PATENT APPLICATION

(11) **EP 1 920 701 A1**
(43) Date of publication of application: **14.05.2008**
(21) Application number: 06746740.7
(22) Date of filing: 23.05.2006
(51) Int. Cl.: A61B 1/00, A61B 19/00

(54) **MEDICAL DEVICE**

(30) Priority: 02.09.2005 JP 2005255272
(71) Applicant: OLYMPUS MEDICAL SYSTEMS CORP., Shibuya-ku Tokyo 151-0072 (JP)
(72) Inventor: KANAZAWA, Noriaki c/o Intellectual Property Support Department, Hachioji-shi Tokyo 192-8512 (JP)
(74) Representative: von Hellfeld, Axel
(86) International application number: PCT/JP2006/310256
(87) International publication number: WO 2007/029380

(57) **Abstract**

A medical apparatus includes a medical device (12), a first support mechanism (142), a second support mechanism (144), and a resistance force generation mechanism (146). The first support mechanism has the medical device arranged therein, and a turning portion (152) that is turned around a predetermined rotation axis. The second support mechanism has the first support mechanism arranged therein and includes a support portion (164) that supports the turning portion to allow the support portion to turn around the rotation axis of the first support mechanism. The resistance force generation mechanism is provided between the turning portion of the first support mechanism and the support portion of the second support mechanism and produces a predetermined resistance force with respect to a relative turning motion of the turning portion and the support portion.

## Description

### Technical Field

The present invention relates to a medical apparatus that supports various kinds of medical device, e.g., an endoscope or an electrocautery.

### Background Art

Jpn. Pat. Appln. KOKAI Publication No. 7-227398 discloses a structure that disposes an insertion portion of an endoscope to a coupling portion of the endoscope. The insertion portion of the endoscope is pressed and fixed with respect to an axial direction of the insertion portion from a lateral side through a slide ring by a spring urging force of a compression spring. It is to be noted that a pressed state of the slide ring can be released by pressing a press button and the pressed state cannot be released unless the press button is pressed.

Jpn. Pat. Appln. KOKAI Publication No. 2002-224016 discloses a holding portion that holds an endoscope while allowing the endoscope to turn. When a force in a twisting direction is applied to an insertion portion, the holding portion can easily rotate the insertion portion.

In the structure that disposes a grip portion of the endoscope disclosed in Jpn. Pat. Appln. KOKAI Publication No. 7-227398, the pressed state from the lateral side of the insertion portion cannot be released unless the press button is pressed. Therefore, when a force is applied in a twisting direction (around an axis of the insertion portion), the pressed state must be released by pressing the press button in order to rotate the insertion portion. That is, when the endoscope is fixed to the holding portion, even if a small twisting force is to be applied to the insertion portion of the endoscope in order to rotate the endoscope, the endoscope cannot be rotated. Furthermore, since one side serves as a fixing end, the force based on the twisting operation is stored on the fixing end side and cannot be released, thus a loop may occur due to the twisting operation, and hence a reaction force is produced when eliminating the loop (releasing the force), thus degrading operability in some cases.

On the other hand, when the endoscope as disclosed in Jpn. Pat. Appln. KOKAI Publication No. 2002-224016 is held in the turnable holding portion, just applying a small force in the twisting direction to the insertion portion of the endoscope causes the insertion portion of the endoscope to turn with respect to the holding portion. That is, the insertion portion of the endoscope easily rotates every time the insertion portion is twisted. Therefore, when trying to restore the insertion portion to its original state (releasing the force in the twisting direction), the reaction force does not correspond to the amount of twisting, thus degrading operability in some cases.

### Disclosure of Invention

In order to solve such a problem, it is an object of the present invention to provide a medical apparatus that can rotate a medical device in accordance with a force in a rotating direction transmitted to the medical device or can restrict the rotation when the medical device is held.

To achieve this object, a medical apparatus according to an aspect of the present invention includes a medical device, a first support mechanism, a second support mechanism, and a resistance force generation mechanism. The first support mechanism has the medical device arranged therein, and a turning portion that is turned around a predetermined rotation axis. The second support mechanism has the first support mechanism arranged therein and includes a support portion that supports the turning portion to allow the support portion to turn around the rotation axis of the first support mechanism. The resistance force generation mechanism is provided between the turning portion of the first support mechanism and the support portion of the second support mechanism and produces a predetermined resistance force with respect to a relative turning motion of the turning portion and the support portion.

### Brief Description of Drawings

FIG. 1 is a schematic view showing a structure of a medical apparatus according to a first embodiment of the present invention;
FIG. 2 is a schematic view showing a detailed structure of the medical apparatus according to the first embodiment;
FIG. 3 is a schematic perspective view showing a base portion, and an insertion portion and a universal cable extended from the base portion in the endoscope according to the first embodiment;
FIG. 4 is a schematic top view showing a structure of an endoscope holding portion used in the medical apparatus according to the first embodiment;
FIG. 5 is a schematic cross-sectional view of the endoscope holding portion used in the medical apparatus according to the first embodiment taken along a line V-V in FIG. 4;
FIG. 6A is a schematic cross-sectional view of the endoscope holding portion used in the medical apparatus according to the first embodiment taken along a line VI-VI in FIG. 4;
FIG. 6B is a schematic perspective view showing a click plate formed at an upper end of an outer ring of the endoscope holding portion used in the medical apparatus according to the first embodiment;
FIG. 7 is a schematic longitudinal sectional view of an endoscope holding portion used in a medical apparatus according to a second embodiment of the present invention;
FIG. 8 is a schematic longitudinal sectional view of an endoscope holding portion used in a medical apparatus according to a third embodiment of the present invention;
FIG. 9A is a schematic cross sectional view of an endoscope holding portion used in a medical apparatus according to a fourth embodiment of the present invention;
FIG. 9B is a schematic cross sectional view of the endoscope holding portion used in the medical apparatus according to the fourth embodiment of the present invention;
FIG. 9C is a schematic cross sectional view of the endoscope holding portion used in the medical apparatus according to the fourth embodiment of the present invention;
FIG. 9D is a schematic cross sectional view of the endoscope holding portion used in the medical apparatus according to the fourth embodiment of the present invention;
FIG. 9E is a schematic cross sectional view of the endoscope holding portion used in the medical apparatus according to the fourth embodiment of the present invention;
FIG. 9F is a schematic cross sectional view of the endoscope holding portion used in the medical apparatus according to the fourth embodiment of the present invention;
FIG. 10A is a schematic cross sectional view of an endoscope holding portion used in a medical apparatus according to a fifth embodiment of the present invention;
FIG. 10B is a schematic cross sectional view of the endoscope holding portion used in the medical apparatus according to the fifth embodiment of the present invention; and
FIG. 11 is a schematic view showing a structure of a medical apparatus according to a sixth embodiment of the present invention.

### Best Mode for Carrying Out the Invention

The best modes for carrying out the present invention will now be explained hereinafter with reference to the accompanying drawings.

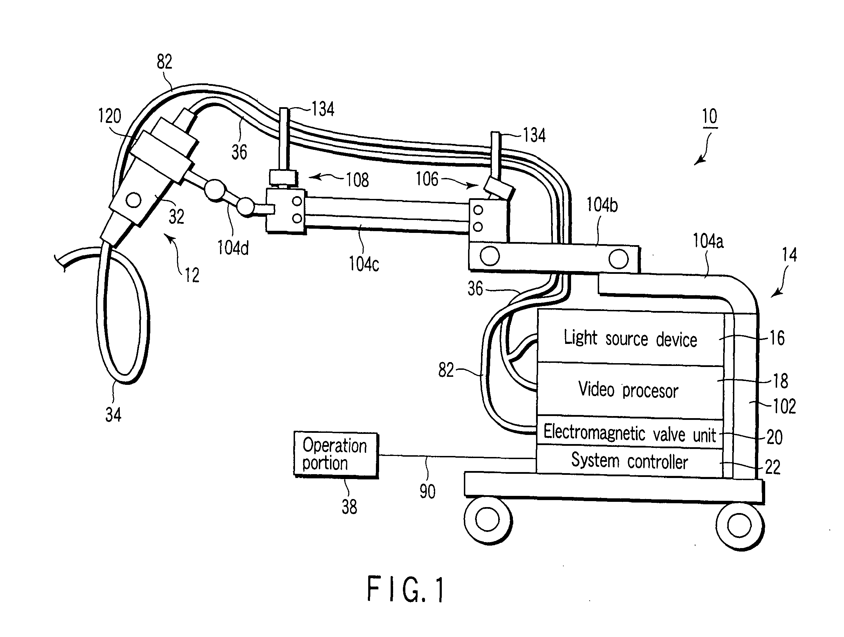

A first embodiment will now be explained with reference to FIGS. 1 to 6B.

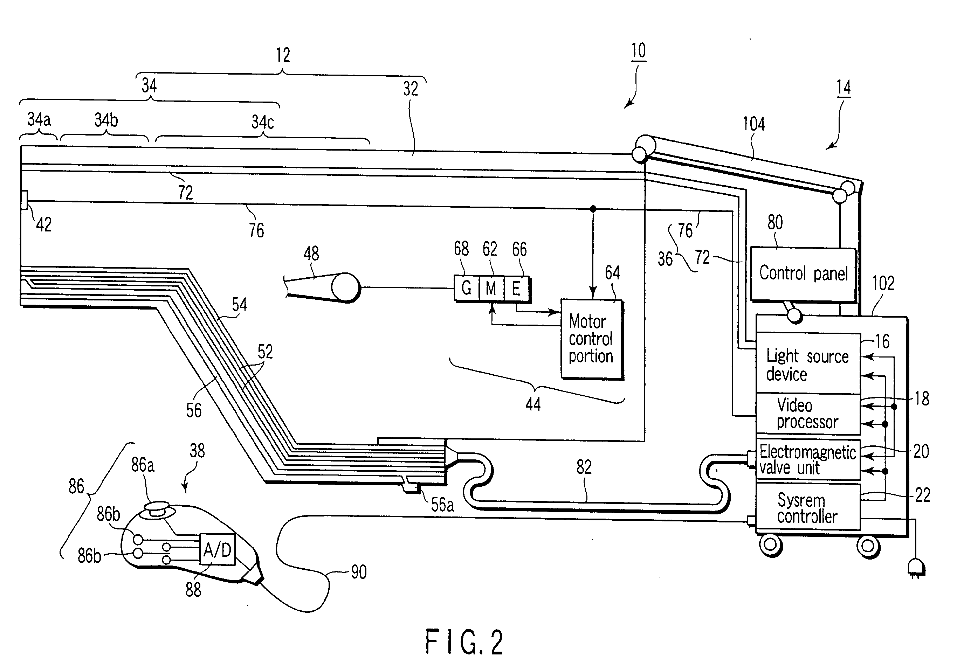

As shown in FIGS. 1 and 2, a medical apparatus 10 according to the embodiment includes an electric bending endoscope (a medical device) 12, a support device 14, a light source device 16, a video processor 18, an electromagnetic valve unit 20, and a system controller 22. The electric bending endoscope 12 has an observing function and a treatment function for a body cavity. The support device 14 supports the electric bending endoscope 12 to allow its movement within a predetermined range. The light source device 16 supplies an illumination light flux exiting from a front surface at a distal end portion of a later-explained insertion portion 34. The video processor 18 receives a video signal from a later-explained imaging unit 42 to perform predetermined signal processing. The electromagnetic valve unit 20 controls air supply/water supply and suction operations through, e.g., a later-explained air supply/water supply duct 52 or suction duct 54 provided in an insertion portion 34. The system controller 22 is electrically connected with the light source device 16, the video processor 18, and the electromagnetic valve unit 20. Therefore, the system controller 22 can control the driving of a later-explained bending driving mechanism 44 and also collectively control the light source device 16, the video processor 18, or the electromagnetic valve unit 20.

As shown in FIG. 2, the endoscope 12 integrally includes a base portion 32 having, e.g., a substantially cylindrical shape or a substantially columnar shape, an elongated insertion portion (a first flexible body) 34 extended from one side surface of the base portion 32, and an elongated universal cable (a second flexible body) 36 extended from the other side surface of the base portion 32.

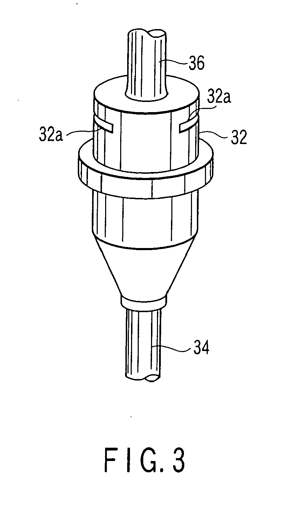

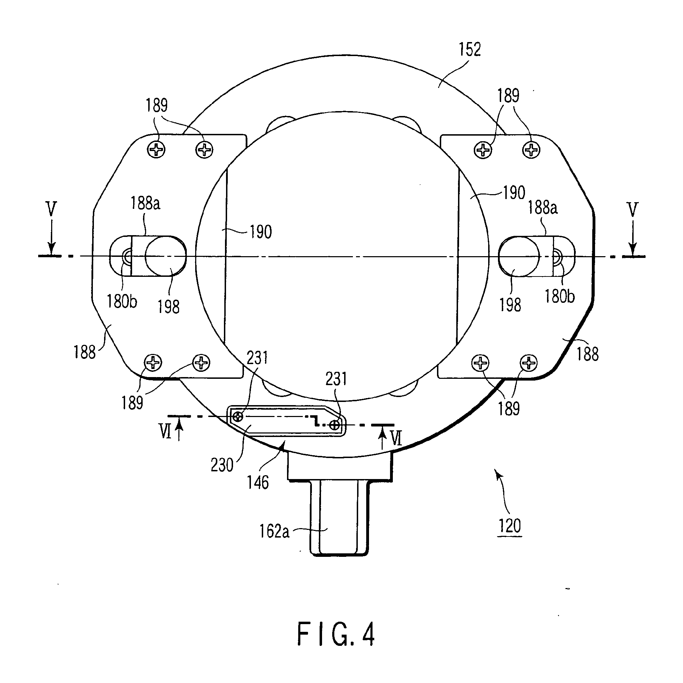

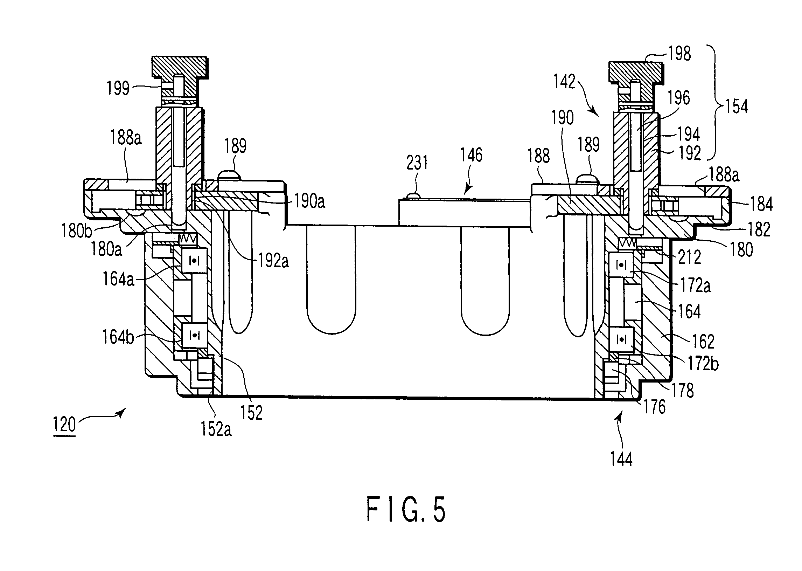

As shown in FIG. 3, a pair of fitting groove portions 32a in which a locking slide plate 190 (see FIGS. 4 and 5) of a later-explained endoscope holding portion 120 (see FIG. 1) are fitted are formed on an outer periphery of the base portion 32.

The insertion portion 34 and the universal cable 36 are arranged on the same axis with respect to the base portion 32. Both the insertion portion 34 and the universal cable 36 have flexibility. As shown in FIGS. 1 and 2, an end portion of the universal cable 36 is optically connected with the light source device 16 and also electrically connected with the video processor 18. The endoscope 12 further includes an operation portion 38 that bends a later-explained bending portion 34b or performs air supply/water supply or suction. The operation portion 38 is electrically connected with the system controller 22.

The insertion portion 34 includes a distal end hard portion 34a formed on the outermost distal end side thereof, the bending portion 34b continuously provided on a proximal end side of the distal end hard portion 34a, and a flexible tube portion 34c continuously provided on a proximal end side of the bending portion 34b and having an elongated shape. The distal end hard portion 34a has a built-in imaging unit 42 formed of, e.g., an imaging device such as an imaging optical system (not shown) or a CCD. The bending portion 34b is configured to bend in upper, lower, left, and right directions by driving control of the later-explained bending driving mechanism 44 that is controlled in response to a bending operation instruction from the operation portion 38.

The base portion 32 has the built-in bending driving mechanism 44 that bends the bending portion 34b. The insertion portion 34 extended from the base portion 32 has flexibility to be inserted into a duct in a body cavity. The light source device 16 and video processor 18 are respectively connected, optically and electrically, with the end portion of the universal cable 36 extended from the other side of the base portion 32.

Moreover, an angle wire 48 that receives a driving force from the driving mechanism 44 to be driven is inserted into the insertion portion 34. Although not shown, the angle wire 48 is connected with the distal end side of the bending portion 34b. Therefore, when the angle wire 48 is driven upon receiving a driving force from the bending driving mechanism 44 of the base portion 32, the bending portion 34b bends in upper, lower, left, and right directions.

The air supply/water supply duct 52 and the suction duct 54 are inserted into the insertion portion 34. An air supply/water supply opening is formed at a distal end of the air supply/water supply duct 52, and a suction opening is formed at a distal end of the suction duct 54. An air supply/water supply opening is formed in the base portion 32 at a proximal end of the air supply/water supply duct 52, and a suction opening is formed in the base portion 32 at a proximal end of the suction duct 54. One end of each tube 82, explained later, is connected with the air supply/water supply opening at the proximal end of the air supply/water supply duct 52 and the suction opening at the proximal end of the suction duct 54. That is, the one end of each tube 82 is connected with the base portion 32. Additionally, a forceps duct 56 into which a treatment instrument, e.g., a forceps is inserted in the insertion portion 34. A forceps opening is formed in a front surface of the forceps duct 56 on a distal end side. A proximal end of the forceps duct 56 communicates with a forceps insertion opening 56a formed at the proximal end portion of the insertion portion 34 near the base portion 32. Therefore, a treatment instrument, e.g., a forceps inserted from the forceps insertion opening 56a can protrude from the distal-end-side front surface of the insertion portion 34 through the forceps duct 56.

The bending driving mechanism 44 is a bending driving means constituted of an electric motor 62 or various kinds of members formed to transmit or disconnect a force produced from the electric motor 62. The bending driving mechanism 44 includes the electric motor 62, a motor control portion 64, an encoder 66, and a reduction gear 68.

The electric motor 62 produces a driving force based on rotation. The motor control portion 64 collectively controls the bending driving mechanism 44 including the electric motor 62. The encoder 66 forms data of an operating state, e.g., a rotation speed or a rotation amount of a driving shaft of the electric motor 62. The reduction gear 68 reduces a rotating force of the driving shaft of the electric motor 62.

A light guide 72 is connected with the light source device 16. The light guide 72 is inserted into the universal cable 36, the base portion 32, and the insertion portion 34 to be extended to the distal end of the insertion portion 34. Therefore, an illumination light flux supplied from the light source device 16 exits the distal end of the insertion portion 34 through the light guide 72.

A signal cable 76 through which a video signal from the imaging unit 42 is transmitted is connected with the video processor 18. The signal cable 76 is extended from the imaging unit 42 at the distal end of the insertion portion 34, and inserted into the insertion portion 34, the base portion 32, and the universal cable 36 to be connected with a predetermined terminal of the video processor 18. Further, a control panel 80 is electrically connected with the video processor 18. A video signal output from the video processor 18 is transmitted to the control panel 80. Upon receiving the signal, a predetermined endoscopic image is displayed in the control panel 80 by using a display portion. Furthermore, in the control panel 80, the display portion and an operation section on a display screen of the display portion are provided. Therefore, various kinds of operation instructions can be input from the operation section.

A pair of tubes (second flexible bodies) 82 communicating with the air supply/water supply duct 52 or the suction duct 54 of the insertion portion 34 are connected with the electromagnetic valve unit 20. That is, the electromagnetic valve unit 20 communicates with the distal end of the insertion portion 34 via the tubes 82, the air supply/water supply duct 52, and the suction duct 54. Therefore, when the electromagnetic valve unit 20 is driven to perform an air supply/water supply operation, air supply/water supply can be effected from a distal facet of the insertion portion 34 through the tubes 82 and the air supply/water supply duct 52 of the base portion 32 and the insertion portion 34. Furthermore, when the electromagnetic valve unit 20 is driven to perform a suction operation, suction can be carried out from the distal facet of the insertion portion 34 through the suction duct 54 of the insertion portion 34 and the base portion 32, and the tubes 82. Moreover, the universal cable 36 is harder to bend as compared with the tubes 82 since the tubes 82 are formed of a hollow flexible resin material but the light guide 72 or the signal cable 76 are arranged in the universal cable 36. That is, the tubes 82 are formed with a lower torque transmission rate than that of the universal cable 36.

The operation portion 38 includes various kinds of operation members that produce a bending operation instruction and air supply/water supply and suction operation instructions, and is separately constituted from the base portion 32. The operation portion 38 includes a multi-operation member 86 and an A/D converter 88. The multi-operation member 86 includes an operation stick 86a that issues a bending operation instruction and an operation button 86b that issues an air supply/water supply operation instruction or a suction operation instruction. The multi-operation members 86a and 86b are electrically connected with the A/D converter 88. Therefore, the A/D converter 88 carries out A/D conversion processing to provide a predetermined operation instruction signal upon receiving an electric signal produced from the multi-operation member 86a or 86b.

The operation portion 38 is electrically connected with the system controller 22 via an electric cable 90. Therefore, various kinds of operation instruction signals generated by the A/D converter 88 when the respective operation members of the operation portion 38 are operated are transmitted to the system controller 22 via the electric cable 90. Furthermore, the light source device 16, the video processor 18, the electromagnetic valve unit 20, and the control panel 80 are respectively electrically connected with the system controller 22. Therefore, upon receiving each of various instruction signals from the operation portion 38, the system controller 22 appropriately transmits a control signal that is used to perform control associated with the instruction signal to each device. Moreover, upon receiving each of various operation instruction signals from the operation portion in the control panel 80, the system controller 22 appropriately transmits a control signal that is used to perform control associated with the instruction signal to each device.

The support device 14 includes a support device base portion 102, an arm 104, and first and second support portions 106 and 108. The support device base portion 102 is a cart formed with, e.g., casters. The support device base portion 102 can freely move on a floor in a state where the light source device 16, the video processor 18, the electromagnetic valve unit 20, the system controller 22, and the control panel 80 are accommodated and mounted therein. The arm 104 supports the endoscope 12 and moves the endoscope 12 within a predetermined range. The first and second support portions 106 and 108 are arranged in, e.g., the arm 104. The first support portion 106 supports the universal cable 36 and the tubes 82, and the second support portion 108 also supports the universal cable 36 and the tubes 82.

As shown in FIG. 1, the arm 104 is supported on the support device base portion 102. The arm 104 includes first to fourth arms 104a, 104b, 104c, and 104d. One end of the first arm 104a is fixed to the support device base portion 102. One end of the second arm 104b is supported at the other end of the first arm 104 to allow its horizontal movement by a pin (not shown) extended in an up-and-down direction (a vertical direction). That is, the second arm 104b is an arm that horizontally moves the endoscope 12. It is to be noted that, for example, a non-illustrated electromagnetic brake is arranged around the pin that supports the other end of the first arm 104a and the one end of the second arm 104b. Therefore, the second arm 104b can be arranged at a desired position with respect to the first arm 104a within a predetermined turning range.

One end of the third arm 104c is supported at the other end of the second arm 104b to allow a vertical movement by a pin (not shown) extended in the horizontal direction. That is, the third arm 104c is an arm that vertically moves the endoscope 12. It is to be noted that, for example, a non-illustrated electromagnetic brake is arranged around the pin that supports the other end of the second arm 104b and the one end of the third arm 104c. Therefore, the third arm 104c can be arranged at a desired position with respect to the second arm 104b within a predetermined turning range.

One end of the fourth arm 104d is supported at the other end of the third arm 104c. The endoscope holding portion 120 is arranged at the other end of the fourth arm 104d. The fourth arm 104d can be inclined with one or more joints since the insertion portion 34 of the endoscope 12 may be held in an inclined state in some cases. Moreover, since a large force may be applied to the joints, arranging an electromagnetic brake at each joint is preferable. Therefore, the endoscope 12 can be fixed at a desired angle within a predetermined range. Additionally, the base portion 32 of the endoscope 12 is supported by the endoscope holding portion 120 to be rotatable around the axis of the insertion portion 34 or the universal cable 36.

For example, an upper surface at one end of the third arm 104c is formed to have a flat surface. The first support portion 106 is fixed to the upper surface at the one end of the third arm 104c. Further, for example, an upper surface at the other end of the third arm 104c is formed to have a flat surface. The second support portion 108 is fixed to the upper surface at the other end of the third arm 104c. These first and second support portions 106 and 108 can turn or rotate with respect to the third arm 104c.

Each of these first and second support portions 106 and 108 includes a bundle retainer 134. For example, the universal cable 36 is arranged in one bundle retainer (a first holding member) 134 of these first and second support portions 106 and 108. For example, the tubes 82 are arranged in the other bundle retainer (a second holding member) 134. These bundle retainers 134 allow movements of the universal cable 36 or the tubes 82 in the axial direction, and also allow periaxial rotation/rotation.

As shown in FIGS. 4 to 6B, the endoscope holding portion 120 includes first and second support mechanisms 142 and 144 and a brake mechanism (a resistance force generation mechanism) 146.

The first support mechanism 142 includes a cylindrical inner ring (a turning portion) 152 in which the base portion 32 of the endoscope 12 is arranged, and a stopper 154 that is attached in a state where the base portion 32 of the endoscope 12 is arranged in the inner ring 152. The second support mechanism 144 includes a cylindrical cover (a coupling portion) 162 connected with the fourth arm 104d, and a cylindrical outer ring (a support portion) 164 arranged on the inner side of the cover 162.

An arm attachment shaft 162a disposed to the fourth arm 104d is formed on the outer periphery of the cover 162. Therefore, the cover 162 is disposed to the distal end of the fourth arm 104d by the arm attachment shaft 162a. The outer ring 164 is fixed on the inner side of the cover 162.

The inner ring 152 is arranged on the inner side of the outer ring 164. A pair of bearings 172a and 172b, are fixed on an inner peripheral surface of the outer ring 164 in an aligned state. That is, the bearings 172a and 172b are arranged between the inner peripheral surface of the outer ring 164 and the outer peripheral surface of the inner ring 152. These bearings 172a and 172b are arranged in concave portions 164a and 164b respectively formed on the inner peripheral surface of the outer ring 164. Further, a male screw portion 152a is formed on the outer peripheral surface at the lower end portion of the inner ring 152. A ring-shaped stopper screw 176 can be screwed to the male screw portion 152a. Therefore, a ring-shaped collar 178 is arranged to push in the lower bearing 172b, and the collar 178 is fixed by the stopper screw 176, thereby fixing the bearing 172b. Therefore, the inner ring 152 can rotate or turn with respect to the outer ring 164 while using a central axis of the inner ring 152 as a spindle. That is, the inner ring 154 and the outer ring 164 have a common central axis.

A flange portion 180 radially outwardly protruding with respect to the central axis of the inner ring 152 is formed at the upper end of the inner ring 152. A pair of first and second concave portions 180a and 180b, in which a lower end portion of a later-explained stopper pin 196 can be arranged, are formed in the flange portion 180. The first concave portion 180a is formed to be closer to the central side of the inner ring 152 than the second concave portion 180b. The first concave portion 180a is formed with a depth almost reaching the flange portion 180. The second concave portion 180b is formed to be shallower than the first concave portion 180a.

As shown in FIG. 5, protruding portions 182 radially outwardly protruding are formed on the flange portion 180 to be symmetrical with respect to the central axis. An edge portion 184 that is upwardly bent in FIG. 5 is formed at an outer end portion of each protruding portion 182.

A stopper 154 is arranged on the flange portion 180. The stopper 154 includes a top plate 188, a slide plate 190, a tube 192, a compression spring 194, the stopper pin 196, and a knob 198.

The top plate 188 is fixed to the flange portion 180 by a screw 189 in a state where it is mounted on the edge portion 184 of the flange portion 180. The slide plate 190 is arranged between a lower surface of the top plate 188 and an upper surface of the flange portion 180. Each slide plates 190 can be inserted into or removed from a space between the lower surface of the top plate 188 and the upper surface of the flange portion 180.

A through hole 188a having a substantially rectangular shape is formed in each top plate 188. A longitudinal direction of each through hole 188a is a radial direction with respect to the central axis of the inner ring 152.

A male screw portion 192a is formed at a lower end portion of the tube 192. Moreover, a through hole is formed in each slide plate 190, and a female screw portion 190a is formed on an inner peripheral surface of the through hole. Therefore, the male screw portion 192a at the lower end portion of the tube 192 is screwed into the female screw portion 190a of the slide plate 190 via the through hole 188a of the top plate 188. That is, the tube 192 is erected from the slide plate 190 via the through hole 188a of the top plate 188. Therefore, the tube 192 can slide in the through hole 188a of the top plate 188. Then, when the tube 192 is moved along the through hole 188a, the slide plate 190 is inserted into or removed from the top plate 188 within a predetermined range.

The compression spring 194 is arranged in the tube 192. Additionally, the stopper pin 196 is arranged in the compression spring 194 in a state where it is pressed toward the lower end portion of the tube 192. The knob 198 is fixed at an upper end portion of the stopper pin 196 by a set screw 199 in a state where the knob 198 upwardly protrudes from the upper end portion of the tube 192.

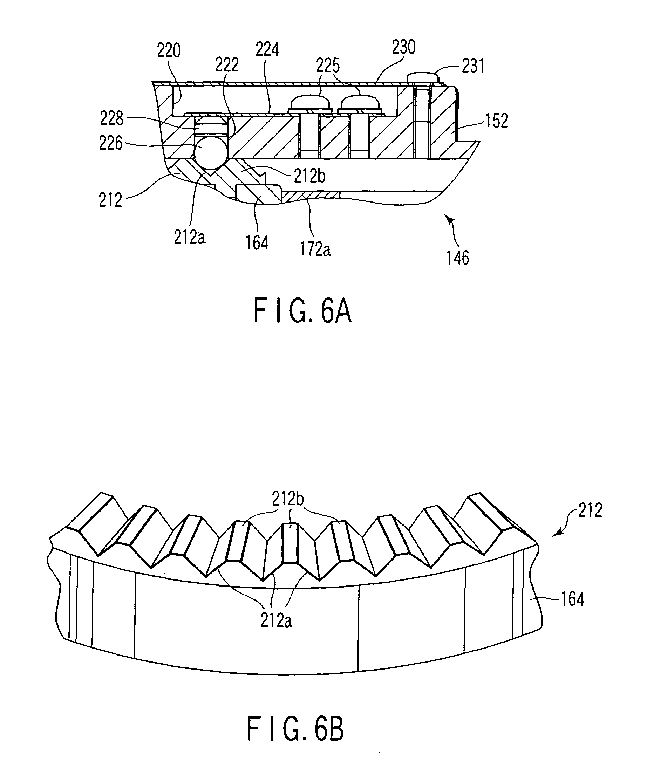

As shown in FIG. 6A, the brake mechanism 146 is provided to inhibit rotation between the inner ring 152 and the outer ring 164.

As shown in FIG. 6B, a click plate (an engagement portion) 212 is integrally fixed on an upper end surface of the outer ring 164. A plurality of V-shaped portions 212a, each having a V-like cross section, and a plurality of chevron portions 212b, each having a chevron cross section, are formed on the click plate 212 to be adjacent to each other. These V-shaped portions 212a and chevron portions 212b are formed over the entire upper surface of the click plate 212. That is, the upper surface of the outer ring 164 is formed in a jagged shape.

A concave portion 220 is formed in the flange portion 180 of the inner ring 152. A through hole 222 is formed in the concave portion 220. A leaf spring (an urging portion) 224 is fixed to the concave portion 220 of the flange portion 180 of the inner ring 152 by a screw 225 to cover the through hole 221 from the upper side. The leaf spring 224 presses a click ball 226 toward the click plate 212 of the outer ring 164 through a later-explained support member 228. It is to be noted that an urging force (a pressing force) for the support member 228 exerted by the leaf spring 224 is appropriately set based on a material, a board thickness, a distance from the screw 225, and other factors.

The click ball (a contact portion) 226 is mounted on one of the V-shaped portions 212a of the click plate 212. A part of the click ball 226 is accommodated in the through hole 222 in the concave portion 220 of the flange portion 180 of the inner ring 152. The support member 228 is arranged between the click ball 226 and the leaf spring 224. The click ball 226 moves in the axial direction of the through hole 222 in a state where a part of the click ball 226 is maintained in the through hole 222 in the concave portion 220 of the inner ring 152.

When the inner ring 152 turns with respect to the outer ring 164, the click ball 226 gradually moves toward the upper side by the chevron portions 212b of the click plate 212, and presses the support member 228 against the leaf spring 224. Therefore, the click ball 226 can move beyond one chevron portion 212b by rotation of the inner ring 152 to be accommodated in the V-shaped portion 212a adjacent to the chevron portion 212b.

It is to be noted that fixing a blind plate 230 to the concave portion 220 through a screw 231 is preferable in order to prevent foreign particles, etc. from entering the concave portion 220 of the inner ring 152.

A function of the medical apparatus 10 according to this embodiment will now be explained.

It is assumed that each slide plate 190 in the endoscope holding portion 120 is in a state depicted in FIGS. 4 and 5. In this state, since each slide plate 190 is arranged on the central axis side apart from the inner peripheral surface of the inner ring 152, the base portion 32 of the endoscope 12 cannot be inserted into the inner ring 152.

Thus, each knob 198 of the stopper 154 is grasped to move each stopper pin 196 toward the upper side in FIG. 5 against the urging force of each compression spring 194. Therefore, an engagement between the lower end of each stopper pin 196 and the second concave portion 180b is released. In this state, the tube 192 is moved toward the radially outer side in the longitudinal direction of the through hole 188a of the top plate 188. That is, the tube 192 of the stopper 154 is moved to the outer end portion from the inner end portion of the through hole 188a. Then, the slide plate 190 is pulled in with respect to the inner peripheral surface of the inner ring 152 to be inserted into the lower side of the top plate 188. In this state, each knob 198 is released. Then, the lower end of the stopper pin 196 is engaged with the first concave portion 180a by the urging force of the compression spring 194. That is, the base portion 32 of the endoscope 12 can be arranged on the inner side of the inner ring 152.

Additionally, the base portion 32 is inserted into the inner side of the inner ring 152 from the upper side or the lower side of the endoscope holding portion 120. At this time, the respective slide plates 190 are fitted into the pair of fitting groove portions 32a of the base portion 32. In this case, each knob 198 is grasped to move each stopper pin 196 to the upper side in FIG. 5 against the urging force of the compression spring 194. Therefore, the engagement between the lower end of the stopper pin 196 and the second concave portion 180b is released. In this state, the tube 192 is moved toward the radially inner side in the longitudinal direction of the through hole 188a of the top plate 188. That is, the tube 192 of the stopper 154 is moved from the outer end portion to the inner end portion of the through hole 188a to protrude the slide plate 190 toward the inner peripheral surface side of the inner ring 152. Each slide plate 190 is inserted into each of the pair of fitting groove portions 32a of the base portion 32 of the endoscope 12. Then, the lower end of the stopper pin 196 is engaged with the first concave portion 180a by the urging force of the compression spring 194. That is, the base portion 32 of the endoscope 12 is disposed on the inner side of the inner ring 152. At this time, since the first concave portion 180a is formed to be sufficiently deeper than the second concave portion 180b, each stopper pin 196 hardly comes off. Therefore, even if a large force is applied to the endoscope holding portion 120 via the base portion 32 of the endoscope 12, a state where each slide plate 190 is inserted into each fitting groove portion 32a of the base portion 32 is maintained.

The endoscope 12 is used in a state where it is held in the endoscope holding portion 120 in this manner.

The distal end portion of the insertion portion 34 of the endoscope 12 depicted in FIGS. 1 and 2 is introduced to a desired position of, e.g., a duct in a body cavity. At this time, besides operating the operation portion 38 to bend the bending portion 34b, an operator may grasp the insertion portion 34 to insert the insertion portion 34 while turning or rotating it around the axis of the insertion portion 34 as one of techniques facilitating insertion. When the insertion portion 34 is turned in this manner, this turning force is transmitted to the base portion 32 from the insertion portion 34. That is, the turning force is transmitted to the base portion 32 held in the endoscope holding portion 120 with the turning motion of the insertion portion 34. Therefore, the turning force is transmitted from the base portion 32 to the inner ring 152 through each slide plate 190.

The inner ring 152 is made to turn with respect to the outer ring 164 held by the cover 162 fixed at the distal end of the fourth arm 104b. In this case, the click ball 226 pressed against both inclined surfaces of the V-shaped portion 212a by the leaf spring 224 through the support member 228 is pressed against one inclined surface of the V-shaped portion 212a by the through hole 222 of the inner ring 152. The click ball 226 is thus made to climb the inclined surface of the V-shaped portion 212a, i.e., an inclined surface of the chevron portion 212b. Then, the click ball 226 presses the leaf spring 224 toward the upper side through the support member 228.

Further, when a predetermined force or a larger force is applied to the inner ring 152, the click ball 226 elastically deforms the leaf spring 224 against its urging force, and passes over the chevron portion 212b. That is, the click ball 226 is accommodated in a neighboring V-shaped portion 212a. If the state where the predetermined force or a larger force is applied to the inner ring 152 is maintained, a function that the click ball 226 further passes over a neighboring chevron portion 212b to be accommodated in a neighboring V-shaped portion 212a is repeated. The inner ring 152 turns with respect to the outer ring 164 as long as application of the predetermined force or a larger force continues.

Incidentally, in a case where the inner ring 152 turns with respect to the outer ring 164, a sound or vibration is produced when the leaf spring 224 comes into contact with the concave portion 220 of the inner ring 152 by the elastic force of the leaf spring 224, or a sound or vibration when the click ball 226 is accommodated in the V-shaped portion 212a is produced. That is, a sense of something fitting into place can be obtained.

On the other hand, even if the predetermined force or a smaller force is applied to the inner ring 152, the click ball 226 pressed toward the lower side by the leaf spring 224 through the support member 228 cannot cross the chevron portion 212b. Therefore, when a predetermined force or a smaller force is applied to the inner ring 152, the inner ring 152 is prevented from turning with respect to the outer ring 164.

Here, the end portion of the universal cable 36 is connected with the light source device 16 and the video processor 18. Further, the end portion of each tube 82 is connected with the electromagnetic valve unit 20. Therefore, the amount the universal cable 36 or each tube 82 can turn is limited. That is, the base portion 32 or the insertion portion 34 is also limited in how much it can be turned. Therefore, when an operator grasps the insertion portion 34 to turn the insertion portion 34, a reaction force that restores this turning motion is produced. On the other hand, the operator keeps exercising the turning force against this reaction force to maintain a state where the insertion portion 34 is turned.

When restoring the turned state of the insertion portion 34 (turning the insertion portion 34 in an opposite direction to be restored to a straight state), the turned state must be slowly restored if the insertion portion 34 is inserted into a body cavity. Here, when restoring the insertion portion 34 to its original turned state from the periaxial turned state, the click ball 226 accommodated in the V-shaped portion 212a repeats the function of crossing the chevron portion 212b while elastically deforming the leaf spring 224 against the urging force of the leaf spring 224 through the support member 228. When the inner ring 152 is turned with respect to the outer ring 164, since the click ball 226 must cross the chevron portion 212b against the urging force of the leaf spring 224, a braking effect that prevents the turned state from being restored occurs. Furthermore, since a reaction energy with respect to the turning motion is consumed every time the click ball 226 sequentially crosses each chevron portion 212b, the turning motion that restores the turned state of the insertion portion 34 to the original turned state is dampened, due to the consumption of the energy.

Although a force is transmitted to the base portion 32 to turn when the insertion portion 34 is turned around its axis, the base portion 32 does not turn in some cases even when the universal cable 36 is turned around its axis and the force is transmitted to the base portion 32. This phenomenon occurs from a difficulty in transmission of the force since the universal cable 36 is formed to be more flexible than the insertion portion 34. Therefore, when a torque allowing the base portion 32 to turn is applied to a root of the universal cable 36, the base portion 32 turns and the insertion portion 34 also turns.

Therefore, according to the endoscope holding portion 120 of this embodiment, although the base portion 32 turns when the insertion portion 34 is turned around its axis, the base portion 32 is not prevented from being turned when the universal cable 36 is turned around its axis, but the base portion 32 turns when the predetermined force or a larger force is applied, and the turning motion of the base portion 32 is stopped when the predetermined force or a smaller force is applied.

As explained above, according to this embodiment, the following effect can be obtained.

In the medical apparatus 10 according to this embodiment, the base portion 32 of the endoscope 12 can be supported at the distal end of the arm 104, and the base portion 32 can be turned in accordance with a force in the rotating direction applied to the base portion 32. That is, only when the predetermined force or a larger force is transmitted to the inner ring 152 through the base portion 32 of the endoscope 12 can the inner ring 152 be turned with respect to the outer ring 164. Therefore, since the endoscope holding portion 120 can prevent the base portion 32 from turning when the insertion portion 34 is slightly twisted, operability of the endoscope 12 can be improved. Furthermore, since the base portion 32 turns by the endoscope holding portion 12 when the insertion portion 34 is largely twisted, a reaction force caused due to twisting can be suppressed. That is, since the brake mechanism 146 is provided between the inner ring 152 and the outer ring 164, the reaction force of the turning motion of the base portion 32 can be reduced.

The click ball 226 is pressed against the V-shaped portion 212a or the chevron portion 212b in the through hole 222 by the leaf spring 224. Therefore, even when the turning force transmitted to the base portion 32 of the endoscope 12 is further transmitted to the inner ring 152, the turning motion of the inner ring 152 with respect to the outer ring 164 can be restricted. That is, the click ball 226 can function as a brake that restricts the turning motion of the inner ring 152 with respect to the outer ring 164.

Therefore, the turning motion or the rotating motion can be restricted by the turning force or the rotating force of the inner ring 152 with respect to the outer ring 164, and the turning motion or the rotating motion can be allowed while restricting the turning motion or the rotating motion. That is, the inner ring 152 can be turned or rotated with respect to the outer ring 164 while braking.

Moreover, since the first concave portion 180a is formed deeper than the second concave portion 180b in the flange portion 180 of the inner ring 152, an engagement state of the stopper pin 196 can be changed. That is, when the base portion 32 of the endoscope 12 is supported by the endoscope holding portion 120, the support portion 32 can be constantly supported.

It is to be noted that an intensity of the braking function can be set low by not only changing the leaf spring 224 but also changing the inclination angles of the inclined surfaces of the V-shaped portion 212a and the chevron portion 212b on the click plate 212. Therefore, when turning the inner ring 152 with respect to the outer ring 164, easiness in turning in one direction can be differentiated from easiness in turning in the other direction by changing the inclination angles of the V-shaped portion 212a and the chevron portion 212b.

Additionally, although the example where the brake mechanism 146 is provided at one position in the endoscope holding portion 120 has been explained in this embodiment, providing this mechanism at a plurality of positions is also preferable.

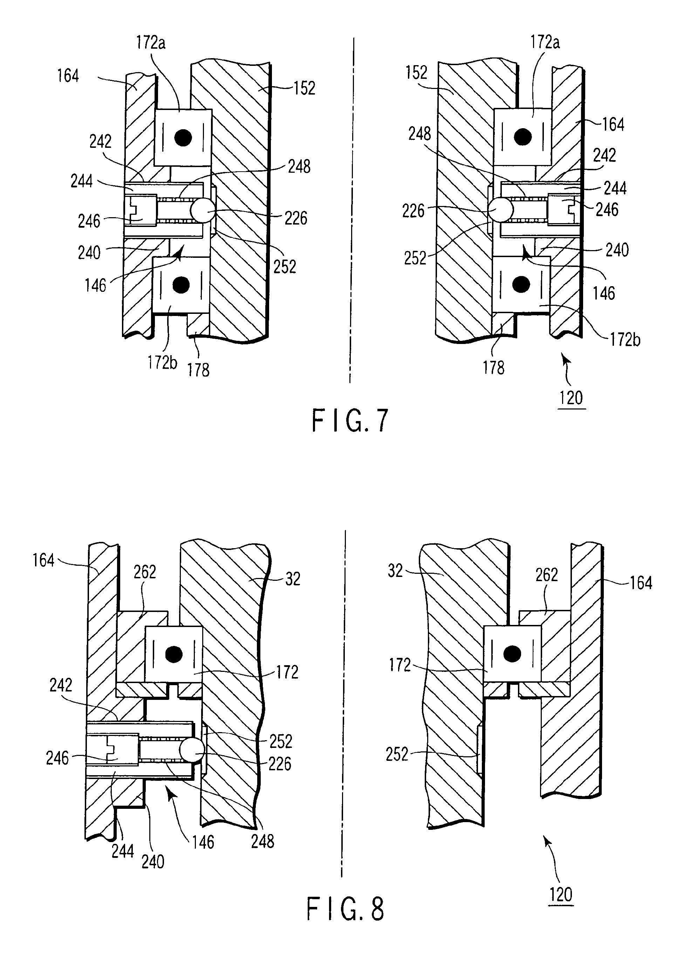

A second embodiment will now be explained with reference to FIG. 7. This embodiment is a modification of the first embodiment, and like reference numerals denote members equal to those explained in the first embodiment or members having the same functions as those explained in the same, thereby omitting a detailed explanation.

In this embodiment, the brake mechanism 146 is modified with respect to the first embodiment.

As shown in FIG. 7, a pair of radially inwardly protruding convex portions 240 are formed on an outer ring 164. That is, the convex portions 240 each partially protruding toward the radially inward direction are formed on an inner peripheral surface of the outer ring 164. These convex portions 240 are formed at, e.g., positions facing a central axis of the outer ring 164. Further, these convex portions 240 are formed between bearings 172a and 172b. A through hole 242 having a female screw portion on an inner peripheral surface thereof is formed in each of these convex portions 240. An axial direction of each of these through holes 242 is a radial direction.

A tube 244 having a male screw portion on an outer peripheral surface thereof is screwed into each of these through holes 242. A female screw portion is formed on an inner peripheral surface of each of these tubes 244. A plunger 246 is screwed and fixed in each of these tubes 244. A click ball 226 is fixed at an end portion of each plunger 246 that is brought into contact with an inner ring 152. Each of these click balls 226 is pressed by a compression spring 248 toward an outer peripheral surface of the inner ring 152. Therefore, each click ball 226 can move closer to or apart from the central axis of the outer ring 164 within a predetermined range.

Concave portions 252 in which the click balls 226 are arranged are formed in the entire outer peripheral surface of the inner ring 152. A step is formed between the concave portions 252 adjacent to each other. The step between the concave portions 252 is formed to have a chevron shape.

A function of an endoscope holding portion 120 of a medical apparatus 10 according to this embodiment will now be explained.

When the inner ring 152 is turned with respect to the outer ring 164, each click ball 226 comes into contact with the step of each concave portion 252 of the inner ring 152. Since the step is formed to have a chevron shape, the click ball 226 crosses the step against an urging force of the plunger 246 to be accommodated in a neighboring concave portion 252 when a turning force is larger than a predetermined turning force. Contrarily, when the turning force is smaller than the predetermined turning force, a state where the click ball 26 is accommodated in the concave portion 252 is maintained.

Therefore, in a case where the inner ring 152 tries turning with respect to the outer ring 164, since the click ball 226 is pressed to the concave portion 252 of the inner ring 152 by the plunger 246 fixed to the outer ring 164, the inner ring 152 turns with respect to the outer ring 164 only when a predetermined force or a larger force is applied. Furthermore, when restoring the turned state, since a certain amount of energy is needed for crossing the step between the concave portion 252 and the adjacent concave portion 252, a braking function occurs.

It is to be noted that the example where the pair of through holes 242 are radially symmetrically provided with respect to the central axis of the outer ring 164 has been explained in this embodiment, though the through holes 242 do not have to be symmetrically provided with respect to the central axis. Moreover, the number of the through holes 242 is not restricted to the pair of the through holes 242, and it may be, e.g., one through hole 242or three through holes 242 to appropriately set the braking function.

Additionally, the example where each click ball 226 or each plunger 246 is provided to the outer ring 164 and each concave portion 252 is provided to the inner ring 152 has been explained in this embodiment. Further, providing each concave portion 252 to the outer ring 164 and providing each click ball 226 or each plunger 246 to the inner ring 152 is also preferable.

Further, simultaneously using the brake mechanism 146 explained in the first embodiment and the brake mechanism 146 described in this embodiment is also preferable.

A third embodiment will now be explained with reference to FIG. 8. This embodiment is a modification of the first and second embodiments, and like reference numerals denote members equal to those explained in the first and second embodiments or members having the same function as those in these embodiments, thereby omitting a detailed explanation thereof.

According to this embodiment, the way in which a base portion 32 of an endoscope 12 is held is modified with respect to the first embodiment.

As shown in FIG. 8, bearing holding portions 262 each holding a bearing 172 are arranged on an outer peripheral surface of the base portion 32 of the endoscope 12. An outer peripheral surface of the bearing holding portion 262 can be attached to/detached from an outer ring 164 in a state where the bearing holding portion 262 is mounted on a convex portion 240 of the outer ring 164.

Therefore, the outer peripheral surface of the base portion 32 of the endoscope 12 can rotate or turn with respect to the outer ring 164. That is, when the base portion 32 of the endoscope 12 is mounted with respect to the outer ring 164 from the upper side, the base portion 32 of the endoscope 12 can be held by an endoscope holding portion 120.

A brake mechanism 146 according to this embodiment is obtained by modifying the brake mechanism 146 explained in the second embodiment. Here, concave portions 266 in which click balls 226 are respectively arranged are formed on the entire outer peripheral surface of the base portion 32 of the endoscope 12. A step is formed between the concave portions 226 adjacent to each other. The step between the concave portions 266 is formed to have a chevron shape.

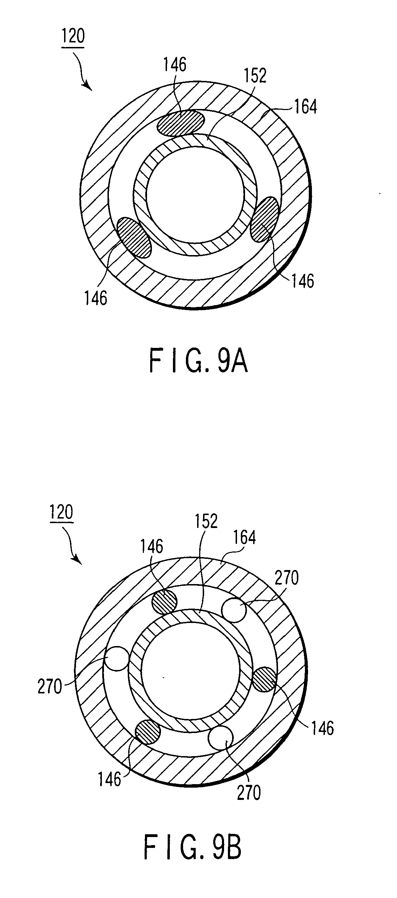

A fourth embodiment will now be explained with reference to FIGS. 9A to 9F. This embodiment is a modification of the first to third embodiments, and like reference numerals denote members equal to those explained in the first to third embodiments or members having the same functions as those explained in these embodiments, thereby omitting a detailed explanation.

As shown in FIG. 9A, brake mechanisms 146 are provided between an outer peripheral surface of an inner ring 152 and an inner peripheral surface of an outer ring 164. Each brake mechanism 146 in this example is an elastic body formed of, e.g., a resin material that produces a frictional force between the outer peripheral surface of the inner ring 152 and the inner peripheral surface of the outer ring 164. Here, for example, the three brake mechanisms 146 are formed at substantially equal intervals. Therefore, the inner ring 152 can be turned or rotated in a desired direction in a state where it is restricted with respect to the outer ring 164.

As shown in FIG. 9B, each brake mechanism 146 is provided between an outer peripheral surface of an inner ring 152 and an inner peripheral surface of an outer ring 164. The brake mechanism 146 may be the same as or different from the brake mechanism 146 depicted in FIG. 9A. Here, each ball bearing 270 is arranged between the outer peripheral surface of the inner ring 152 and the inner peripheral surface of the outer ring 164. The brake mechanism 146 restricts rotation between the inner ring 152 and the outer ring 164 to produce a resistance force. Therefore, a rotating or turning motion between the outer peripheral surface of the inner ring 152 and the inner peripheral surface of the outer ring 164 is restricted.

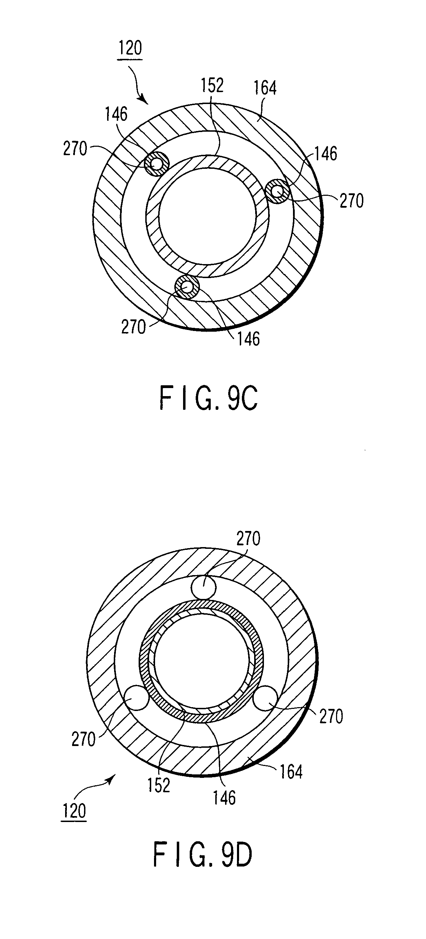

As shown in FIG. 9C, each brake mechanism 146 is provided between an outer peripheral surface of an inner ring 152 and an inner peripheral surface of an outer ring 164. The brake mechanism 146 restricts rotation of each ball bearing 270 to produce a resistance force. Therefore, a rotating or turning motion between the outer peripheral surface of the inner ring 152 and the inner peripheral surface of the outer ring 164 is restricted.

As shown in FIG. 9D, a brake mechanism 146 is provided on an outer peripheral surface of an inner ring 152. Each ball bearing 270 is arranged between the brake mechanism 146 and an inner peripheral surface of an outer ring 164. The brake mechanism 146 restricts rotation of each ball bearing 270 to produce a resistance force. Therefore, a rotating or turning motion between the outer peripheral surface of the inner ring 152 and the inner peripheral surface of the outer ring 164 is restricted.

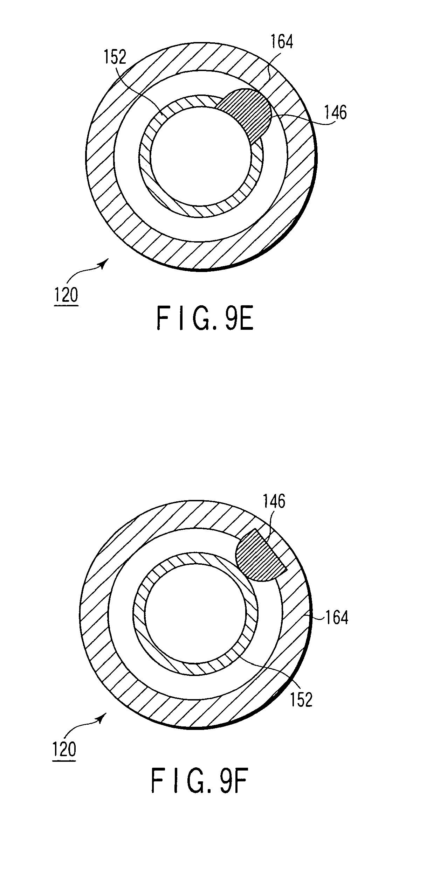

As shown in FIG. 9E, a brake mechanism 146 is provided to an inner ring 152. The brake mechanism 146 has, e.g., a tongue-like shape, and its distal end is in contact with an inner peripheral surface of an outer ring 164. Therefore, a rotating or turning motion between an outer peripheral surface of the inner ring 152 and the inner peripheral surface of the outer ring 164 is restricted.

As shown in FIG. 9F, a brake mechanism 146 is provided to an outer ring 164. The brake mechanism 146 has, e.g., a tongue-like shape, and its distal end is in contact with an outer peripheral surface of an inner ring 152. Therefore, a rotating or turning motion between the outer peripheral surface of the inner ring 152 and an inner peripheral surface of the outer ring 164 is restricted.

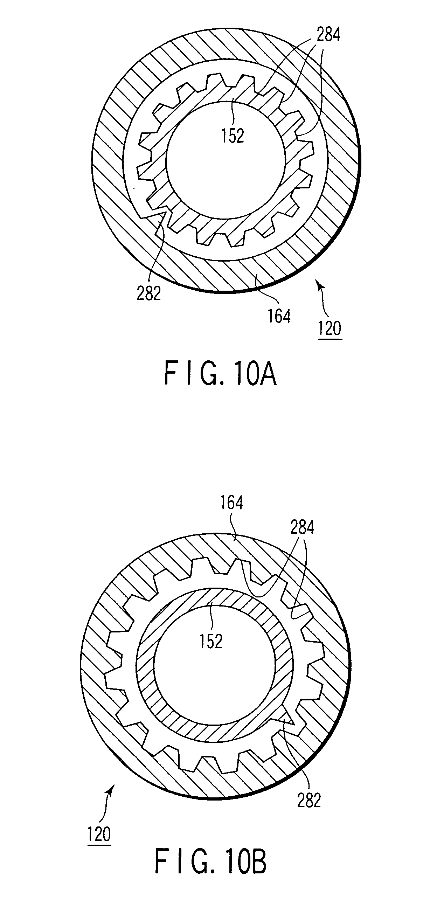

A fifth embodiment will now be explained with reference to FIGS. 10A and 10B. This embodiment is a modification of the first to fourth embodiments, and like reference numerals denote members equal to those explained in the first to fourth embodiments or members having the same functions as those explained in these embodiments, thereby omitting a detailed explanation thereof.

As shown in FIG. 10A, a brake mechanism 146 includes a protruding portion 282 as an elastic body formed on an inner peripheral surface of an outer ring 164 and concave portions 284 formed on an entire outer peripheral surface of an inter ring 152 at predetermined intervals. The protruding portion 282 provided on the inner peripheral surface of the outer ring 164 is accommodated in one of the concave portions 284 of the inner ring 152.

When the inner ring 152 turns with respect to the outer ring 164, the protruding portion 282 of the outer ring 164 is brought into contact with an edge portion of the concave portion 284 of the inner ring 152 in which the protruding portion 282 of the outer ring 164 is accommodated. When a turning force of the inner ring 152 is equal to or above a predetermined force, the edge portion of the concave portion 284 elastically deforms and pushes away the protruding portion 282 of the outer ring 164 to be accommodated in an adjacent concave portion 284. On the other hand, when the turning force is equal to or below the predetermined force, the turning motion of the inner ring 152 is restricted by the edge portion of the concave portion 284.

Further, as shown in FIG. 10B, a structure that is opposite to the structure depicted in FIG. 10A is also preferable. In this case, concave portions 284 are formed on an entire inner peripheral surface of an outer ring 164 at predetermined intervals. On the other hand, a protruding portion 282 as an elastic body is formed on an outer peripheral surface of an inner ring 152.

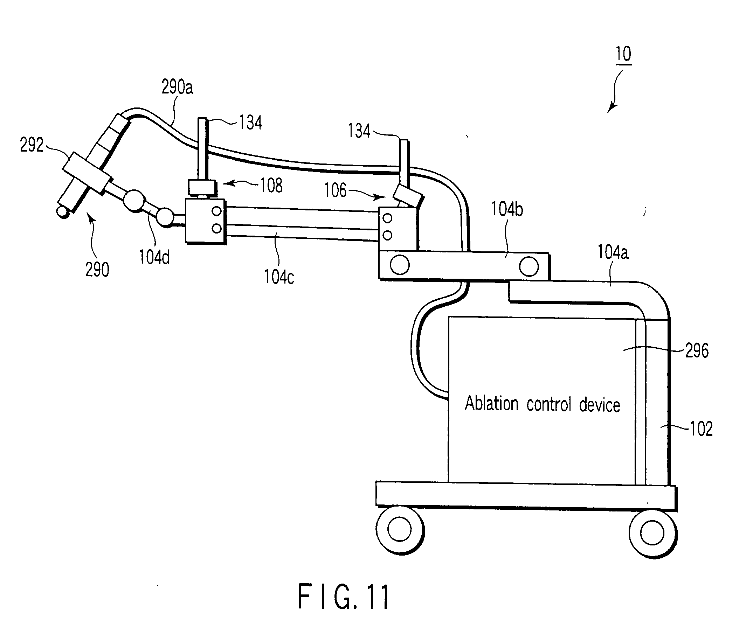

A sixth embodiment will now be explained with reference to FIG. 11. This embodiment is a modification of the first to fifth embodiments, and like reference numerals denote members equal to those explained in the first to fifth embodiments or members having the same functions as those explained in these embodiments, thereby omitting a detailed description thereof.

As shown in FIG. 11, in this example, an electrocautery holding portion 292 that holds a electrocautery 290 having a cable 290a is formed at a distal end of a fourth arm 104d in place of an endoscope holding portion 120. An ablation control device 296 is connected with an end portion of the cable 290a. This ablation control device 296 is supported on a support device base portion 102.

A structure of the electrocautery holding portion 292 in this example is substantially the same as, e.g., the holding portion 292 explained in the third embodiment. That is, the holding portion 292 or 120 can be appropriately changed in accordance with a shape and others of a medical device, e.g., the electrocautery 290 or the endoscope 12.

Although the several embodiments have been specifically explained with reference to the drawings, the present invention is not restricted thereto and includes all embodiments carried out without departing from the scope of the invention.

### Industrial Applicability

According to the present invention, it is possible to provide the medical apparatus that can allow or restrict rotation of a medical device in accordance with a force in a rotating direction transmitted to the medical device when the medical device is held.

## Claims

1. A medical apparatus (10) **characterized by** comprising:
a medical device (12);
a first support mechanism (142) which has the medical device arranged therein and includes a turning portion (152) that is turned around a predetermined rotation axis;
a second support mechanism (144) which has the first support mechanism arranged therein and includes a support portion (164) that supports the turning portion to allow the support portion to turn around the rotation axis of the first support mechanism; and
a resistance force generation mechanism (146) which is provided between the turning portion of the first support mechanism and the support portion of the second support mechanism and produces a predetermined resistance force with respect to a relative turning motion of the turning portion and the support portion.

2. The medical apparatus (10) according to claim 1, **characterized by** comprising:
a first flexible body (34) which is arranged in the medical device (12) and extended from the medical device in a predetermined direction; and
a second flexible body (36) which is arranged in the medical device and extended from the medical device in a direction different from that of the first flexible body,
wherein the second flexible body has a lower torque transmission rate than that of the first flexible body.

3. The medical apparatus (10) according to claim 2, **characterized in that** the second flexible body (36) is more flexible than the first flexible body (34).

4. The medical apparatus (10) according to claim 1, **characterized in that**
the resistance force generation mechanism (146) includes:
a contact portion (226) which is provided to the turning portion (152) and comes into contact with the support portion (164); and
an urging portion (224) that urges the contact portion with respect to the support portion with a predetermined force amount.

5. The medical apparatus (10) according to claim 4, **characterized in that** the support portion (164) includes a plurality of engagement portions (212) that is able to engage with the contact portion (226) in a turning direction of the turning portion (152).

6. The medical apparatus (10) according to claim 1, **characterized in that**
the resistance force generation mechanism (146) includes:
a contact portion (226) that is provided to the support portion (164) and comes into contact with the turning portion (152); and
an urging portion (224) which urges the contact portion with respect to the turning portion with a predetermined force amount.

7. The medical apparatus according to claim 6, **characterized in that** the turning portion (152) includes a plurality of engagement portions (212) which is able to engage with the contact portion (226) in a turning direction of the turning portion.

8. The medical apparatus (10) according to claim 1, **characterized in that**
the support portion (164) is formed to have a cylindrical shape,
the turning portion (152) includes a flange portion (180) which faces a cylindrical facet of the support portion, and
the resistance force generation mechanism (146) is provided between the facet of the support portion and the flange portion.

9. A medical apparatus (10) **characterized by** comprising:
an inner ring (152) in which a medical device (12) is arranged on an inner side thereof;
an outer ring (164) in which the inner ring is arranged on an inner side thereof to allow the inner ring to turn or rotate; and
a resistance force generation mechanism (146) which restricts a turning or rotating motion of the inner ring with respect to the outer ring when this motion has a predetermined turning or rotating force or a smaller force, and allows the turning or rotating motion while restricting the same when this motion has the predetermined turning or rotating force or a larger force.

10. The medical apparatus (10) according to claim 9, **characterized in that** the medical device (12) is integral with the inner ring (152).
